# EUROPEAN PATENT APPLICATION

(11) **EP 4 484 541 A1**
(43) Date of publication of application: **01.01.2025**
(21) Application number: 23760105.9
(22) Date of filing: 24.02.2023
(51) Int. Cl.: C12M 1/00, C12M 1/12, C12M 1/26, C12N 1/02

(54) **CELL SEPARATION APPARATUS AND CELL SEPARATION METHOD**

(30) Priority: 25.02.2022 JP 2022028240
(71) Applicant: Fujimori Kogyo Co., Ltd., Tokyo 112-0002 (JP)
(72) Inventor: TOKURA, Tomohiro, Tokyo 112-0002 (JP); MATSUDA, Hiroyuki, Tokyo 112-0002 (JP)
(74) Representative: Lavoix
(86) International application number: PCT/JP2023/006743
(87) International publication number: WO 2023/163107

(57) **Abstract**

This cell separation apparatus (10) is an apparatus that processes a suspension containing cells and microcarriers to separate the cells and the microcarriers including: a separation device (11) that separates the microcarriers from the cells by a filter medium, a supply path (12) that supplies the suspension to the separation device (11), a recovery path (13) that recovers the suspension containing the cells after the microcarriers have been removed by the separation device (11), a pressure sensor (15) that detects a pressure of the suspension, and a control unit (16) that controls the supply of the suspension through the supply path (12) in response to a change in pressure detected by the pressure sensor (15).

## Description

### TECHNICAL FIELD

The present invention relates to a cell separation apparatus and a cell separation method.

Priority is claimed on Japanese Patent Application No. 2022-028240, filed February 25, 2022, the content of which is incorporated herein by reference.

### BACKGROUND ART

Mass culture of microorganisms, insect cells, plant cells, animal cells, and the like is widely used for the production of various substances useful as pharmaceuticals, foods, cosmetics, and raw materials for these. For example, when culturing cells that are adhesion-dependent (anchorage-dependent), such as totipotent stem cells derived from multicellular organisms, it may be necessary to provide a scaffold to replace other cells or extracellular matrix.

In an adhesion culture method in which cells are attached to a culture surface of a culture container or the like, there is a problem that the cell yield is low due to the constraints of the culture area. In a suspension culture method in which cells are suspended in a culture medium, it is possible to increase the yield depending on the volume of the culture medium. However, in order to suppress excessive cell aggregation in the culture medium, complex control is required, such as applying a shear force to the culture medium by stirring to a degree that does not cause cell death.

A microcarrier culture method that uses a scaffold material (microcarrier) that can be suspended in the culture medium has been proposed as a culture method that combines the advantages of both adhesion culture method and suspension culture method. In this method, cells can be suspended in the culture medium while being attached to the scaffold material. Therefore, it is easier to control the culture of adhesion-dependent cells.

However, in the microcarrier culture method, since the microcarriers are mixed together, this may hinder the use of the cultured cells. Therefore, a step of detaching the cells from the microcarriers and a step of removing the microcarriers from the cell containing suspension are required after culturing the cells. For example, Patent Documents 1 and 2 propose cell separation apparatuses that include a container partitioned by a porous mesh or net-like structure.

### CITATION LIST

### Patent Document

[Patent Document 1]
   Published Japanese Translation No. 2018-537993
[Patent Document 2]
   PCT International Publication No. WO 2020/054755

### SUMMARY OF INVENTION

### Technical Problem

When the culture medium containing cells is filtered using a cell separation apparatus that uses a filter medium such as a porous mesh or net-like structure, the microcarriers and the cells can be separated. However, for example, when a large amount of microcarriers adhere to the filter medium, cells are difficult to pass through the filter medium, and cell recovery may stagnate. Furthermore, if the pressure of the culture medium increases during filtration due to clogging of the filter medium, the cells may be damaged.

The present invention has been made in view of the above-described circumstances and an object of the present invention is to provide a cell separation apparatus and a cell separation method capable of efficiently separating cells and microcarriers.

### Solution to Problem

In order to solve the above-described problems, each aspect of the present invention has the following configuration.
[Aspect 1] Aspect 1 is a cell separation apparatus for processing a suspension containing cells and microcarriers to separate the cells and the microcarriers, including: a separation device that separates the microcarriers from the cells by a filter medium; a supply path that supplies the suspension to the separation device; a recovery path that recovers the suspension containing the cells after the microcarriers have been removed by the separation device; a pressure sensor that detects a pressure of the suspension; and a control unit that controls the supply of the suspension through the supply path in response to a change in pressure detected by the pressure sensor.
[Aspect 2] Aspect 2 is the cell separation apparatus of Aspect 1, wherein the pressure sensor is disposed in the supply path.
[Aspect 3] Aspect 3 is the cell separation apparatus of Aspect 1 or 2, wherein in the separation device, the suspension flows upward with respect to the filter medium.
[Aspect 4] Aspect 4 is the cell separation apparatus of any one of Aspects 1 to 3, wherein the recovery path includes a path that supplies a backwashing liquid from the recovery path to the separation device.
[Aspect 5] Aspect 5 is the cell separation apparatus of Aspect 4, wherein the supply path includes a path that recovers the backwashing liquid after backwashing of the separation device.
[Aspect 6] Aspect 6 is the cell separation apparatus of any one of Aspects 1 to 5, wherein a plurality of the separation devices are installed in parallel between the supply path and the recovery path.
[Aspect 7] Aspect 7 is a cell separation method of processing a suspension containing cells and microcarriers to separate the cells and the microcarriers, including: controlling the supply of the suspension through a supply path in response to a change in pressure detected by a pressure sensor using a separation device that separates the microcarriers from the cells by a filter medium, the supply path that supplies the suspension to the separation device, a recovery path that recovers the suspension containing the cells after the microcarriers have been removed by the separation device, and the pressure sensor that detects a pressure of the suspension.

### ADVANTAGE OF INVENTION

According to each aspect of the present invention, since the supply of the suspension to the separation device through the supply path is controlled in response to a change in pressure detected by the pressure sensor, it is possible to efficiently separate cells and microcarriers.

### BRIEF DESCRIPTION OF DRAWINGS

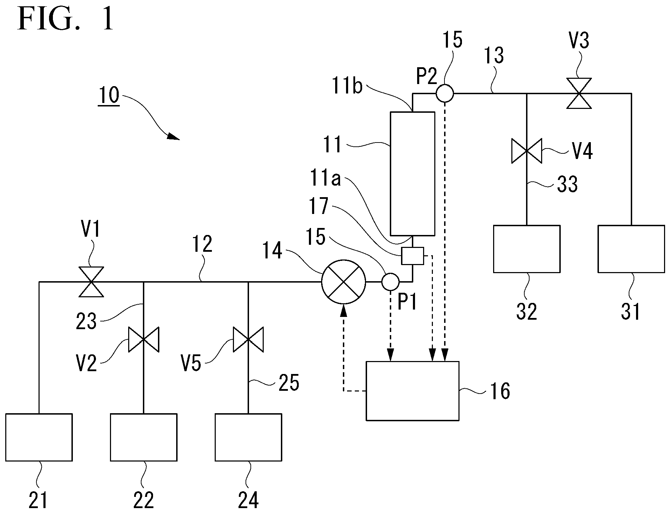

FIG. 1 is a schematic view showing an example of a cell separation apparatus of an embodiment.

### DESCRIPTION OF EMBODIMENTS

Hereinafter, the present invention will be described based on preferred embodiments.

For example, in the culture medium obtained by culturing cells using microcarriers, the cells are suspended in a state where they are attached to the microcarriers. By adding a cell detachment agent such as a proteolytic enzyme or a non-enzymatic detachment agent to the culture medium, the adhesion of the cells to the microcarriers can be dissociated, and the cells can be suspended in the liquid. The suspension obtained in this way contains cells and microcarriers. The cell separation apparatus of the embodiment can process the suspension subjected to the cell detachment process to separate the cells from the microcarriers.

In the following description, the suspension containing cells and microcarriers before the process by a separation device 11 may be referred to as the "pre-separation suspension." Furthermore, the suspension containing cells after the microcarriers have been removed by the separation device 11 may be referred to as the "post-separation suspension." When simply referring to the "suspension," the suspension may include the pre-separation suspension, the post-separation suspension, and the suspension being processed in the separation device 11.

FIG. 1 shows an example of a cell separation apparatus 10 of an embodiment. The cell separation apparatus 10 generally includes the separation device 11 that separates microcarriers from cells using a filter medium, a supply path 12 that supplies the pre-separation suspension to the separation device 11, and a recovery path 13 that recovers the post-separation suspension after microcarriers have been removed by the separation device 11.

The method of sending the suspension through the supply path 12 and the recovery path 13 is not particularly limited, but for example, it is preferable to provide a pump 14 on the side of the supply path 12 and to pressurize the pre-separation suspension from the supply path 12. Since the flow rate of the suspension is increased by pressurizing the pre-separation suspension, the process time can be shortened. As other liquid sending methods, the suspension can be made to flow down due to its own weight or can be suctioned from the recovery path 13.

The separation device 11 includes a filter medium to separate microcarriers from cells in the suspension. The filter medium is capable of blocking the passage of microcarriers while allowing the passage of cells. Although the microcarriers may be trapped by the filter medium, it is preferable that the microcarriers be recoverable away from the filter medium.

The separation device 11 includes an inlet 11a connected to the supply path 12 and an outlet 11b connected to the recovery path 13. The plurality of separation devices 11 may be installed in parallel between the supply path 12 and the recovery path 13.

The flow direction from the inlet 11a to the outlet 11b is not particularly limited, but may be upward, downward, sideways, obliquely upward, or obliquely downward. The flow direction inside the separation device 11 may be uniform. For example, the flow direction inside the separation device 11 may change, for example, in a polygonal line shape, a spiral shape, a crank shape, a radial shape, or the like.

Since gravity acts along the direction in which the cells and liquid in the suspension flow when the flow in the separation device 11 is downward, sideways, or obliquely downward, the kinetic energy required to move the cells can be suppressed. However, microcarriers that are blocked by the filter medium are likely to become trapped by the filter medium.

If the flow in the separation device 11 is upward or obliquely upward, kinetic energy needs to be imparted to the suspension to maintain the flow. However, since gravity acts against the direction in which the cells and liquid in the suspension flow, the microcarriers trapped by the filter medium tend to separate from the filter medium and settle on the side of the inlet 11a of the separation device 11. Accordingly, the clogging of the microcarriers can be suppressed.

In order to facilitate separation of the microcarriers from the filter medium, the flow direction of the suspension in the separation device 11 may be upward relative to the filter medium. For example, the filter medium may be arranged so that the surface through which the suspension flows into the filter medium faces downward or obliquely downward. The flow direction in the separation device 11 may be once guided downward from the inlet 11a and then guided upward toward the filter medium. Furthermore, the flow that is directed upward when passing through the filter medium may be guided downward toward the outlet 11b.

Even when the flow in the separation device 11 is upward, the amount of microcarriers trapped by the filter medium may increase, so that the liquid containing cells is not likely to pass through the filter medium. In addition, if the flow stagnates for some reason and the flow rate of the liquid passing through the filter medium decreases compared to the flow rate of the suspension supplied from the supply path 12 to the separation device 11, the pressure in the supply path 12 may increase.

If the pressure in the supply path 12 increases, the cells in the suspension being supplied through the supply path 12 may be damaged. Alternatively, since the pressure may increase in the space on the side of the supply path 12 of the filter medium even in the separation device 11, a phenomenon similar to that in the supply path 12 may occur. Alternatively, if the microcarriers clog the filter medium and narrow the passages in the filter medium, the cells may be damaged as they pass through the narrow passages. Alternatively, the cells may be subjected to pressure such that they are unable to pass through narrowed passages in the filter medium and may be damaged.

If cells are damaged by a change in pressure or the like, the cells may lose function or even die depending on the extent of the damage. Some cell types are sensitive to mechanical stress and can only tolerate relatively low pressures. Since the consequences of cell damage are often irreversible, it is preferable to remove factors that cause cell damage in advance.

### <Pressure sensor>

The cell separation apparatus 10 of the embodiment includes a pressure sensor 15 that detects the pressure of the suspension. In the illustrated cell separation apparatus 10, the pressure sensor 15 disposed in the supply path 12 is set as a first pressure sensor P1, and the pressure sensor 15 disposed in the recovery path 13 is set as a second pressure sensor P2. Although not specifically shown, the pressure sensor 15 may be disposed on the side of the inlet 11a of the filter medium of the separation device 11 or on the side of the outlet 11b of the filter medium.

For example, the first pressure sensor P1 can observe a situation in which the pressure in the supply path 12 increases as the amount of microcarriers trapped in the filter medium increases. From the viewpoint of suppressing damage to cells, it is preferable to set a threshold value, such as an allowable upper limit value, for the pressure in the supply path 12. For example, the threshold value may be a pressure that is higher than the normal pressure in the supply path 12 before the filter medium becomes clogged and is lower than the pressure at which cell damage becomes noticeable.

In the second pressure sensor P2, when the pressure on the side of the recovery path 13 increases similarly to the pressure on the side of the supply path 12, an allowable upper limit value may be set as a threshold value for the pressure on the recovery path 13. For example, the threshold value may be a pressure that is higher than the normal pressure in the recovery path 13 before the filter medium becomes clogged and is lower than the pressure at which cell damage becomes noticeable. When the pressure on the side of the recovery path 13 decreases due to pressure loss in the filter medium or the like, an allowable lower limit value for the pressure in the recovery path 13 may be set as a threshold value.

The type of the pressure sensor 15 is not particularly limited, but examples include a diaphragm type, a capacitance type, a diffusion type, a resistance wire type, and a piezoresistance type. In order to measure the pressure of the suspension containing cells or microcarriers, it is preferable that the pressure sensor 15 has a pressure detection portion that can be sterilized, washed, or replaced. In order to prevent suspended matter such as cells or microcarriers from entering the pressure sensor 15, an inlet and an outlet may be provided for the pressure detection portion to introduce only the fluid portion of the suspension into the pressure sensor 15.

### <Control unit>

The cell separation apparatus 10 of the embodiment has a control unit 16 for controlling the supply of the suspension in response to a change in pressure. The control unit 16 controls the supply of the pre-separation suspension through the supply path 12 in response to a change in pressure detected by at least one pressure sensor 15 selected from the first pressure sensor P1 and the second pressure sensor P2.

The control unit 16 may include a processing device such as a controller to control the equipment based on information received from sensors such as the pressure sensor 15. The control unit 16 may include a storage device, an input device, an output device, a processing program, and the like. By using the control unit 16, automatic control becomes possible. The supply of the suspension can be controlled manually using the pressure sensor 15. The method of transmitting and receiving the control signal is not particularly limited, and may be wireless or wired.

The method of controlling the supply of the pre-separation suspension in response to a change in pressure is not particularly limited as long as it is a method that responds to a change in pressure. However, examples thereof include a method of stopping the supply of the suspension, a method of reducing the flow rate of the suspension, a method of reducing the flow velocity of the suspension, and the like. From the viewpoint of suppressing the influence on the cells, the flow rate of the suspension may be gradually reduced and the supply of the suspension may be stopped depending on the situation. If the inappropriate pressure change continues, a notification function may be provided, such as issuing an alarm to an operator or the like to have the cell separation apparatus 10 inspected.

While the supply of the suspension is being reduced, it is possible that the pressure will tend to return to normal as the microcarriers come off the filter medium. In such a case, the flow rate of the suspension may be increased to a normal supply rate in the cell separation process without stopping the supply of the suspension.

When the flow of cells in the separation device 11 is upward or obliquely upward, the function of the filter medium can be restored by allowing the microcarriers to settle. Therefore, excess microcarriers may be discharged from the lower portion of the separation device 11 or the like after the supply of the suspension is stopped or suppressed.

The flow rate of the suspension may be reduced and the supply may be stopped by controlling, for example, the operation of the pump 14 or by controlling the valve V1 provided in the supply path 12. The valve V1 is a valve for controlling a flow path from a suspension container 21 storing the pre-separation suspension to the supply path 12. The valve V1 may have a function of selecting the flow of the pre-separation suspension from two states, that is, an open state and a closed state, and may have a function of changing the flow rate of the pre-separation suspension stepwise or continuously.

The second pressure sensor P2 may be omitted, and the supply of the suspension may be controlled in response to a change in pressure detected by the first pressure sensor P1. The first pressure sensor P1 may be omitted, and the supply of the suspension may be controlled in response to a change in pressure detected by the second pressure sensor P2. The supply of the suspension may be controlled in response to a change in pressure detected by the first pressure sensor P1 and the second pressure sensor P2.

### <Washing function>

The supply path 12 may include a washing liquid path 23 for supplying a washing liquid in addition to the path for supplying the pre-separation suspension from the suspension container 21.

The washing liquid is not particularly limited, but it is preferable to use a solution suitable as a dispersion medium for dispersing cells. An appropriate buffer liquid (buffer) can be used as the washing liquid. The washing liquid may contain components of the liquid medium or the like. The washing liquid is preferably a liquid that does not contain cells, and more preferably does not contain cells or microcarriers. However, it is also possible to use a washing liquid that contains microcarriers.

The illustrated washing liquid path 23 merges with the supply path 12 from a washing liquid container 22 that stores the washing liquid. It is preferable that a valve V2 for controlling the flow of the washing liquid is disposed in the washing liquid path 23. The valve V2 may have a function of selecting the flow of the washing liquid from two states, that is, an open state and a closed state, may have a function of changing the flow rate of the washing liquid stepwise or continuously.

During the supply of the pre-separation suspension from the suspension container 21 to the separation device 11, it is preferable to separate the washing liquid path 23 from the supply path 12 by, for example, setting the valve V2 to a closed state. During the supply of the washing liquid to the separation device 11, it is preferable to set the valve V2 in an open state and the valve V1 leading to the suspension container 21 to a closed state.

The washing liquid is preferably supplied to the supply path 12 after the pre-separation suspension is supplied to the supply path 12 and processed in the separation device 11. As a result, if there are any cells remaining in the middle of the separation device 11, the supply path 12, the recovery path 13, and the like, the cells can be washed away with the washing liquid and recovered by processing them in the same way as the suspension.

When the cells and microcarriers remain in the supply path 12, the microcarriers are washed away together with the cells by the washing liquid. At this time, the washing liquid becomes a suspension containing cells and microcarriers. By passing the washing liquid containing the cells and microcarriers through the separation device 11 as with the pre-separation suspension, the microcarriers can be separated and the washing liquid containing the cells can be recovered as with the post-separation suspension.

Although not specifically shown, the suspension container 21 can be removed and replaced with the washing liquid container 22 after the pre-separation suspension is supplied from the suspension container 21 to the supply path 12 and processed by the separation device 11. In this case, the supply path 12 can also serve as the washing liquid path 23 from the position connected to the washing liquid container 22.

Instead of connecting the washing liquid path 23 to the middle of the supply path 12, it is also possible to connect the washing liquid path 23 directly to the separation device 11. In this case, it is not possible to wash the residues in the supply path 12, but it is possible to wash the residues in the separation device 11. Further, it is also possible to supply the washing liquid to the supply path 12 or the separation device 11 using an instrument such as a syringe.

### <Cell recovery>

In the illustrated cell separation apparatus 10, the recovery path 13 includes a cell recovery container 31 that stores the post-separation suspension. The cell recovery container 31 may store or supply substances that contribute to the survival and preservation of the recovered cells.

In the recovery path 13, a valve V3 may be disposed at a desired position between the separation device 11 and the cell recovery container 31. The valve V3 is a valve that controls the flow path from the recovery path 13 to the cell recovery container 31. The valve V3 may have a function of selecting the flow of the post-separation suspension from two states, that is, an open state and a closed state, and may have a function of changing the flow rate of the post-separation suspension stepwise or continuously.

During the supply of the pre-separation suspension or the washing liquid to the separation device 11, the valve V3 is set to an open state. After the cell separation process is completed, it is preferable to set the valve V3 to a closed state. Furthermore, for example, in the event of an abnormality in the separation device 11 or the supply path 12, the valve V3 may be temporarily closed in order to avoid adverse effects on the cells recovered in the cell recovery container 31.

### <Backwashing function>

The recovery path 13 may include a backwashing liquid supply path 33 that supplies the backwashing liquid from the recovery path 13 to the separation device 11.

The backwashing liquid is not particularly limited, but it is preferable to use an appropriate liquid in consideration of the possibility that the backwashing liquid may come into contact with the cells or that the backwashing liquid may remain in the separation device 11 after the backwashing operation. For example, an appropriate buffer liquid (buffer) can be used as the backwashing liquid. The backwashing liquid may contain components of the liquid medium or the like.

The backwashing liquid preferably does not contain microcarriers, and more preferably does not contain cells or microcarriers. The backwashing liquid may have the same composition as the washing liquid or may have a different composition. The backwashing liquid may contain the same components as the washing liquid or may contain different components.

The illustrated backwashing liquid supply path 33 merges with the recovery path 13 from a backwashing liquid supply container 32 that stores the backwashing liquid. It is preferable that a valve V4 for controlling the flow of the backwashing liquid is disposed in the backwashing liquid supply path 33. The valve V4 may have a function of selecting the flow of the backwashing liquid from two states, that is, an open state and a closed state, and may have a function of changing the flow rate of the backwashing liquid stepwise or continuously.

During the supply of the pre-separation suspension or the washing liquid to the separation device 11, it is preferable to separate the backwashing liquid supply path 33 from the recovery path 13 by, for example, setting the valve V4 to a closed state. When performing backwashing, it is preferable to open the valve V4 and close the valve V3 leading to the cell recovery container 31.

In the backwashing using the backwashing liquid, the backwashing liquid is supplied to the filter medium from the outlet 11b of the separation device 11. By flowing the backwashing liquid in the opposite direction to the pre-separation suspension in the cell separation process, the microcarriers or cells attached to the filter medium or the like can be released from the filter medium.

When the cell separation process is performed in a closed atmosphere, it is preferable to guide the backwashing liquid supplied to the separation device 11 to an appropriate container and recover the liquid. For example, the supply path 12 preferably includes a backwashing liquid recovery path 25 for recovering the backwashing liquid after backwashing of the separation device 11 is performed.

In the illustrated cell separation apparatus 10, a backwashing liquid recovery container 24 is provided through the backwashing liquid recovery path 25 branching off from the supply path 12. The backwashing liquid caused to flow back from the inlet 11a of the separation device 11 to the supply path 12 is recovered in the backwashing liquid recovery container 24 from the outlet 11b.

By closing the valves V1 and V2 and then recovering the backwashing liquid in the backwashing liquid recovery container 24, the influence of backwashing on the suspension container 21 or the washing liquid container 22 can be avoided. Further, if the cells received in the backwashing liquid recovery container 24 are in good condition, the cells can be used again in the cell separation process, thereby contributing to the cell yield.

It is preferable that a valve V5 for controlling the flow of the backwashing liquid is disposed in the backwashing liquid recovery path 25. The valve V5 may have a function of selecting the flow of the backwashing liquid from two states, that is, an open state and a closed state, and may have a function of changing the flow rate of the backwashing liquid stepwise or continuously.

During the supply of the pre-separation suspension or the washing liquid to the separation device 11, it is preferable to separate the backwashing liquid recovery path 25 from the supply path 12 by, for example, setting the valve V5 to a closed state. When performing backwashing, the valve V5 is opened and the valves V1 and V2 are closed.

Although not specifically shown, it is also possible to connect the backwashing liquid supply path 33 directly to the separation device 11 instead of connecting the backwashing liquid supply path 33 to the middle of the recovery path 13. By supplying the backwashing liquid from the side of the outlet 11b of the separation device 11 in relation to the filter medium, the filter medium can be backwashed. Further, it is also possible to supply the backwashing liquid to the recovery path 13 or the separation device 11 using an instrument such as a syringe.

Also, it is also possible to connect the backwashing liquid recovery path 25 directly to the separation device 11 instead of connecting the backwashing liquid recovery path 25 to the middle of the supply path 12. By discharging the backwashing liquid from the inlet 11a of the separation device 11 in relation to the filter medium, the backwashing liquid after backwashing of the filter medium can be recovered.

### <Air bubble detection function>

The supply path 12 may include an air bubble detection sensor 17 which detects bubbles of the suspension. If air bubbles are present in the pre-separation suspension, the air bubbles may become trapped in the filter medium to thereby cause the flow of the suspension through the filter media to stagnate. When the air bubble detection sensor 17 detects air bubbles, it is preferable to control the supply of the pre-separation suspension through the supply path 12 regardless of the change in pressure detected by the pressure sensor 15.

The air bubble detection method of the air bubble detection sensor 17 is not particularly limited, but a non-contact method such as an optical method or an ultrasonic method is preferable. The presence or absence of air bubbles can be detected by irradiating inspection light or ultrasonic wave from the outside of a flow path formed by a tube or the like and detecting the difference between when air bubbles pass through the flow path and when liquid passes through the flow path.

The method of controlling the supply of the pre-separation suspension in response to the detection of air bubbles is not particularly limited, but examples thereof include a method of stopping the supply of the suspension, a method of reducing the flow rate of the suspension, a method of reducing the flow velocity of the suspension, and the like. From the viewpoint of suppressing the influence on the cells, the flow rate of the suspension may be gradually reduced and the supply of the suspension may be stopped depending on the situation. If the detection of air bubbles continues, a notification function may be provided, such as issuing an alarm to an operator or the like to have the cell separation apparatus 10 inspected.

The air bubble detection sensor 17 may be installed on the side closer to the suspension container 21 than the position where the washing liquid path 23 merges with the supply path 12 or on the side closer to the separation device 11 than the position where the washing liquid path 23 merges with the supply path 12, or may be installed in the washing liquid path 23. By installing the air bubble detection sensor 17 near the inlet 11a of the separation device 11, even if air bubbles are generated in the supply path 12, the air bubbles can be detected before they flow into the separation device 11.

### <Detail of cell separation process>

The detail of the separation device 11 is not particularly limited, but examples of the filter medium include sheet-shaped mesh, tube-shaped mesh, bag-shaped mesh, and mesh made by weaving wires into a net shape. A large number of through holes may be formed in a film, sheet, tube, or the like to form a mesh.

The material of the filter medium is not particularly limited, but examples thereof include polyolefins such as polyethylene and polypropylene, polyamides such as polyester and nylon, and metals such as stainless steel. Flexible or rigid filter medium may be used. At least during the cell separation process, it is preferable that the filter medium is fixed to the container that contains the filter medium in the separation device 11. It is preferable that the separation device 11 has a replaceable filter medium.

The inlet 11a and the outlet 11b of the separation device 11 may be on the same straight line, the central axis of the inlet 11a and the central axis of the outlet 11b may be shifted in parallel, or the inlet 11a and the outlet 11b may be in different directions. The inlet 11a may be divided into two or more, and the outlet 11b may be divided into two or more. The shape of the separation device 11 near the inlet 11a may be tapered such that the cross-sectional area becomes wider from the inlet 11a. The shape of the separation device 11 near the outlet 11b may be tapered such that the cross-sectional area becomes narrower toward the outlet 11b.

The microcarriers are not particularly limited, but it is preferable that the microcarriers are capable of suspending in the culture medium. The microcarriers may be capable of suspending in the culture medium without stirring or may obtain kinetic energy capable of suspending depending on the stirring speed. The method of stirring the culture medium is not particularly limited, but shaking culture may be used. The specific gravity of the microcarrier may be smaller than or larger than the specific gravity of the culture medium. The microcarrier may be porous, a capsule, a hollow fiber, or the like.

The microcarriers may be fibrous scaffold materials such as nanofibers or particulate scaffold materials such as microbeads. The ratio of the major axis to the minor axis (aspect ratio) of the fibrous scaffold materials may be, for example, 2 or more, about 2 to 500, or about 10 to 300. The particulate scaffold materials may have an aspect ratio of, for example, 2 or less, or about 1 to 1.5. The particle size of the microcarrier is not particularly limited, but may be appropriately selected, for example, from about 0.1 µm to 10 mm.

The material of the microcarrier is not particularly limited, but examples thereof include polysaccharides such as cellulose, hemicellulose, chitin, chitosan, dextran, agarose, and alginate; proteins such as collagen and gelatin; synthetic polymers such as polystyrene, polyacrylamide, polycarbonate, polyethylene, polypropylene, and silicone; and inorganic materials such as glass, silica, and alumina.

The surface of the microcarrier may be coated with a coating material depending on the type of cell, culture conditions, or the like. Examples of the coating material include proteins such as collagen, gelatin, and keratin. In the case of the microcarrier made of a polymer, functional groups such as amino groups, epoxy groups, carboxyl groups, and formyl groups may be imparted by chemically processing the surface of the polymer. The microcarriers may contain a magnetic material to magnetically control the suspended state of the microcarriers.

The cell detachment agent is not particularly limited, but examples thereof include enzymes such as trypsin, protease, collagenase, dispase, and nuclease; chelating compounds such as ethylenediaminetetraacetic acid (EDTA) and nitrilotriacetic acid (NTA); and the like. The cell detachment agent may be composed of one substance or two or more substances. The cell detachment process may be performed within the culture container or after removing the cells and microcarriers from the culture container.

The configuration of each container used in the cell separation apparatus 10 is not particularly limited, and may be a hard bottle, tank, or the like, or a flexible bag, pouch, or the like. Here, examples of the containers include a container storing the filter medium in the separation device 11, the suspension container 21, the washing liquid container 22, the backwashing liquid recovery container 24, the cell recovery container 31, the backwashing liquid supply container 32, but are not limited to these.

The shape of the container is not particularly limited, but examples thereof include a columnar shape, a conical shape, a prismatic shape, a pyramidal shape, a flat shape, a spherical shape, and the like. The material of the container is not particularly limited, but examples thereof include resin, rubber, elastomer, metal, glass, and the like. When the container is flexible, the container may be supported by a hard outer container.

The material of the flexible container is not particularly limited, but examples thereof include polyolefins such as polyethylene (PE) and polypropylene (PP), polyesters such as polystyrene (PS) and polyethylene terephthalate (PET), and polyamides such as nylon. The material of the outer container is not particularly limited, but examples thereof include resin, rubber, elastomer, metal, glass, and the like.

Each container may be provided with an inlet, an outlet, an air supply pipe, an exhaust pipe, a sensor, a temperature control device, and the like. The capacity of the container is not particularly limited, and can accommodate a desired scale, from a small scale for testing to a large scale for production.

The configuration of each flow path used in the cell separation apparatus 10 is not particularly limited, and may be a flexible tube, a hose, or the like, or a hard pipe or the like. The materials for each flow path include resin, rubber, elastomer, metal, and the like. The flow path may be provided with appropriate devices such as a filter, a flow monitor, a flow meter, and a temperature controller. Examples of the flow paths include the supply path 12, the recovery path 13, the washing liquid path 23, the backwashing liquid recovery path 25, and the backwashing liquid supply path 33, but are not limited to these.

At a portion where the container and the flow path are connected or at a portion where the flow path such as a tube is connected in series or in parallel midway through the flow path, a connecting device such as a port or a connector may be used. It is preferable to use a sterile connection port, a sterile connector, or the like that allows a sterile connection.

The configuration of the valve provided in the flow path is not particularly limited, but can be appropriately selected from the viewpoints of operability, reliability, convenience, processing capacity, and the like. For flow paths such as flexible tubes and hoses, a pinch valve may be used that compresses the flow path from the outside to close the flow path. Examples of the valves include the above-described valves V1, V2, V3, V4, and V5, but are not limited to these.

The configuration of the pump provided in the flow path is not particularly limited, but can be appropriately selected from the viewpoints of operability, reliability, convenience, processing capacity, and the like. For the flow path such as a flexible tube or hose, a tube pump may be used that applies power to the liquid contained therein by compressing the flow path from the outside.

The culture of cells or the like to be cultured is not particularly limited, but examples thereof include fungi, bacteria, viruses, yeast, algae, insect cells, plant cells, and animal cells. The cultured cells may be cell lines such as CHO cells, HeLa cells, and COS cells; stem cells such as embryonic stem cells (ES cells), induced pluripotent stem cells (iPS cells), mesenchymal stem cells, and neural stem cells; differentiated tissue cells; or the like.

Cell culture methods include batch culture, fed-batch culture, and perfusion culture. Suspension culture using microcarriers is performed under conditions where the cells and microcarriers are not attached to the culture container, but are attached to the microcarriers. The medium used for suspension culture is preferably a liquid medium. Appropriate ingredients such as salts, sugars, and polysaccharides may be added to the medium.

The cells separated from the microcarriers using the cell separation apparatus 10 of the embodiment are preferably cells cultured using the microcarriers. The cell separation apparatus 10 can also be used for the purpose of separating cells from a suspension containing microcarriers different from those being cultured or for the purpose of separating cells from a suspension containing suspended matter other than microcarriers.

The cells to be subjected to the cell separation process may be cells of a multicellular organism or cells of a unicellular organism. The cells released from the microcarriers may be released individually or may form a cluster containing a plurality of cells.

The containers, flow paths, and the like used in the cell separation process are sterilized as necessary. The sterilization method is not particularly limited, but examples thereof include a radiation sterilization method by irradiation with gamma rays, electron beams, X-rays, or the like, a gas sterilization method using ethylene oxide, hydrogen peroxide, or the like, a hot water immersion sterilization method, a hot water shower sterilization method, and a high-pressure steam sterilization (autoclave) method.

Although the present invention has been described based on the preferred embodiments, the present invention is not limited to the above-described embodiments, and various modifications can be made without departing from the gist of the present invention. Modifications include additions, substitutions, omissions, and other changes to components in each embodiment.

### INDUSTRIAL APPLICABILITY

According to each aspect of the present invention, since the supply of the suspension to the separation device through the supply path is controlled in response to a change in pressure detected by the pressure sensor, it is possible to efficiently separate cells and microcarriers. Therefore, the present invention is industrially applicable.

### REFERENCE SIGNS LIST

V1, V2, V3, V4, V5 Valve
10 Cell separation apparatus
11 Separation device
11a Inlet
11b Outlet
12 Supply path
13 Recovery path
14 Pump
15 Pressure sensor
16 Control unit
17 Air bubble detection sensor 21 Suspension container 22 Washing liquid container 23 Washing liquid path 24 Backwashing liquid recovery container 25 Backwashing liquid recovery path 31 Cell recovery container 32 Backwashing liquid supply container 33 Backwashing liquid supply path

## Claims

1. A cell separation apparatus for processing a suspension containing cells and microcarriers to separate the cells and the microcarriers, comprising:
a separation device that separates the microcarriers from the cells by a filter medium;
a supply path that supplies the suspension to the separation device;
a recovery path that recovers the suspension containing the cells after the microcarriers have been removed by the separation device;
a pressure sensor that detects a pressure of the suspension; and
a control unit that controls the supply of the suspension through the supply path in response to a change in pressure detected by the pressure sensor.

2. The cell separation apparatus according to claim 1,
wherein the pressure sensor is disposed in the supply path.

3. The cell separation apparatus according to claim 1,
wherein in the separation device, the suspension flows upward with respect to the filter medium.

4. The cell separation apparatus according to claim 1,
wherein the recovery path includes a path that supplies a backwashing liquid from the recovery path to the separation device.

5. The cell separation apparatus according to claim 4,
wherein the supply path includes a path that recovers the backwashing liquid after backwashing of the separation device.

6. The cell separation apparatus according to claim 1,
wherein a plurality of the separation devices are installed in parallel between the supply path and the recovery path.

7. A cell separation method of processing a suspension containing cells and microcarriers to separate the cells and the microcarriers, comprising:
using a separation device that separates microcarriers from cells by a filter medium, a supply path that supplies the suspension to the separation device, a recovery path that recovers the suspension containing the cells after the microcarriers have been removed by the separation device, and a pressure sensor that detects a pressure of the suspension, and
controlling the supply of the suspension through the supply path in response to a change in pressure detected by the pressure sensor.
